# EUROPÄISCHE PATENTANMELDUNG

(11) **EP 0 923 925 A2**
(43) Veröffentlichungstag der Anmeldung: **23.06.1999**
(21) Anmeldenummer: 98250426.8
(22) Anmeldetag: 04.12.1998
(51) Int. Cl.: A61K 6/083

(54) **Polymerisierbares Kompositmaterial**

(30) Priorität: 15.12.1997 DE 19757645
(71) Anmelder: IVOCLAR AG, 9494 Schaan (LI)
(72) Erfinder: Rheinberger, Volker, Dr., 9490 Vaduz (LI); Salz, Ulrich, Dr., 88131 Lindau (DE); Höland, Wolfram, Prof. Dr., 9494 Schaan (LI); Rumphorst, André Dr., 9490 Vaduz (LI); Grabher, Kurt, 6800 Feldkirch (AT); Fischer, Urs Karl, 9320 Arbon (CH); Schweiger, Marcel Dipl.-Ing., 7000 Chur (CH); Moszner, Norbert Prof. Dr., 9492 Eschen (LI)
(74) Vertreter: UEXKÜLL & STOLBERG

(57) **Zusammenfassung**

Es wird ein Kompositmaterial beschrieben, welches polymerisierbare Monomere sowie ein spezielles transparentes Glas mit hoher Calcium- und Fluorionen-Freisetzung aufweist und aufgrund dieser Freisetzung Eigenschaft sowie insbesondere seiner optischen Eigenschaften als Restaurationsmaterial in der Zahnheilkunde eingesetzt werden kann.

## Beschreibung

Die Erfindung betrifft polymerisierbares Kompositmaterial, welches ein spezielles transparentes Glas mit hoher Calcium- und Fluorionenabgabe enthält. Das Kompositmaterial ist aufgrund seiner vorteilhaften Eigenschaften insbesondere als Dentalmaterial einsetzbar.

In der Zahnheilkunde besteht nach wie vor ein großes Bedürfnis, die nach Einsatz von dentalem Restaurationsmaterial, wie z.B. eines Füllungskomposites, häufig auftretende Sekundärkaries zu verhindern. Aus diesem Grunde sind in den letzten Jahren auch Füllungskomposite untersucht worden, die in der Mundhöhle Ionen, wie z.B. Fluor-, Calcium- oder Hydroxyl-Ionen freisetzen können. Diese Ionen sind vorteilhaft, da von ihnen eine remineralisierende, bioaktive bzw. kariostatische Wirkung ausgeht.

Restaurative Dentalmaterialien, die durch Gehalt an Fluoridquellen, wie speziellen Chlorhexidin-Fluorid-Verbindungen, eine karieshemmende Wirkung entfalten, sind z.B. aus U. Salz, Phillip Journal 14 (1997) 296 bekannt.

Weitere Beispiele für ionenfreisetzende Füllungsmaterialien sind Glasionomerzemente oder Kompomere, deren organische Matrix stets aus Monomeren, Oligomeren oder Polymeren mit Carboxylgruppen aufgebaut ist (vgl. u.a.: A.D. Wilson, J.W. McLean, Glass Ionomer Cement, Quintessence Publishers, Chicago, 1988; J. Nicholson, M. Anstice, Trends Polym. Sci. **2** (1994) 272; R. Hickel, L. Kremers, C. Haffner, Quintessenz 47 (1996) 1581).

Diese Füllungsmaterialien zeigen zwar einen hohen Grad an Ionenfreisetzung, bei ihnen tritt jedoch nach längerem Kontakt mit Wasser eine deutliche Abnahme der mechanischen Eigenschaften, insbesondere der Festigkeit, auf.

Weiter ist aus der EP-B-449 399 ein dentales Kompositmaterial bekannt, welches als Unterfüllungsmaterial oder als Zement eingesetzt wird. Das Kompositmaterial enthält neben üblichen (Meth)acrylaten ein spezielles Glas, welches Calcium- und Hydroxyl-Ionen freisetzt. Das Glas hat jedoch eine zu hohe Opazität, so daß es dem Kompositmaterial ein unnatürlich totes Aussehen verleiht und daher nicht im Bereich ästhetisch anspruchsvoller Restaurationen eingesetzt werden kann. Die geringe Transluzenz behindert zudem eine Lichthärtung des Kompositmaterials, so daß eine gerade bei Füllungsmaterialien für tiefe Kavitäten erforderliche hohe Durchhärtungstiefe nicht erzielt werden kann. Schließlich enthält das Glas auch nur sehr geringe Mengen an Fluor, welches von ggf. als Flußmittel eingesetztem Kryolith, NaF oder KF stammen kann.

Der Erfindung liegt demgemäß die Aufgabe zugrunde, ein polymerisierbares Kompositmaterial zur Verfügung zu stellen, welches neben einer hohen Calciumionenabgabe auch eine hohe Fluorionen-abgabe bei Kontakt mit Wasser zeigt, durch Licht selbst in tiefen Schichten vollständig ausgehärtet wird und eine hohe Transluzenz aufweist, die seinen Einsatz als Werkstoff für ästhetisch ansprechende Dentalrestaurationen ermöglicht.

Diese Aufgabe wird überraschenderweise durch das polymerisierbare Kompositmaterial nach den Ansprüchen 1 bis 9 gelöst. Die Erfindung betrifft ebenfalls das transparente Glas gemäß Anspruch 10 sowie die Verwendung des Kompositmaterials nach den Ansprüchen 11 und 12.

Das erfindungsgemäße polymerisierbare Kompositmaterial zeichnet sich dadurch aus, daß es
(a) mindestens ein polymerisierbares Monomer und
(b) mindestens ein transparentes Glas mit hoher Calcium- und Fluorionenabgabe aufweist, welches die folgenden Komponenten enthält:

| Komponente | Gew.-% |
|---|---|
| | |
| SiO₂ | 24,0 bis 56,0 |
| CaO | 26,0 bis 57,0 |
| F | 4,0 bis 14,0. |

Das erfindungsgemäß eingesetzte Glas enthält bevorzugt zusätzlich mindestens eine der folgenden Komponenten

| Komponente | Gew.-% |
|---|---|
| | |
| Na₂O | 1,0 bis 9,0 |
| B₂O₃ | 1,0 bis 14,0 |
| MgO | 1,0 bis 14,0 |
| SrO | 1,0 bis 12,0 |
| ZnO | 1,0 bis 7,0 |
| Al₂O₃ | 0,5 bis 5,0 |
| ZrO₂ | 0,5 bis 4,0. |

Für die einzelnen Komponenten des Glases existieren bevorzugte Mengenbereiche. Diese können unabhängig voneinander gewählt werden und sind wie folgt

| Komponente | Gew.-% |
|---|---|
| | |
| SiO₂ | 30,0 bis 54,0, insbesondere 36,0 bis 54,0 |
| CaO | 32,0 bis 50,0 |
| F | 5,0 bis 12,0 |
| Na₂O | 1,0 bis 8,0 |
| B₂O₃ | 1,0 bis 12,0 |
| MgO | 1,0 bis 10,0 |
| SrO | 1,0 bis 10,0 |
| ZnO | 1,0 bis 5,0 |
| Al₂O₃ | 0,5 bis 4,0 |
| ZrO₂ | 0,5 bis 4,0. |

Besonders bevorzugte Mengenbereiche der Komponenten des Glases, die unabhängig voneinander gewählt werden können, sind wie folgt

| Komponente | Gew.-% |
|---|---|
| | |
| SiO₂ | 45,0 bis 54,0 |
| CaO | 35,0 bis 50,0 |
| F | 6,0 bis 12,0 |
| Na₂O | 4,0 bis 7,0 |
| B₂O₃ | 1,0 bis 12,0 |
| MgO | 1,0 bis 10,0 |
| SrO | 1,0 bis 10,0 |
| ZnO | 1,0 bis 5,0 |
| Al₂O₃ | 0,5 bis 4,0 |
| ZrO₂ | 0,5 bis 4,0. |

Alle vorstehend sowie im folgenden in der Beschreibung und in den Ansprüchen angegebenen Mengen der Komponenten des Glases sind als Werte zu verstehen, die wie folgt erhalten wurden: Die Mengen der Oxide wurden durch quantitative Bestimmung der entsprechenden Kationen, d.h. Si, Ca, Na, B, Mg, Sr, Zn, Al und Zr, mittels Röntgenfluoreszenz-Analyse und Umrechnung der erhaltenen Werte in die Mengen an den jeweiligen Oxiden ermittelt. Somit wird von dem Gehalt eines Kations auf den Gehalt des entsprechenden Oxides geschlossen. Demgegenüber wird die Menge an F direkt mittels einer für Fluoridionen selektiven Elektrode bestimmt, nachdem das Glas einem Soda-Potasche-Aufschluß unterzogen worden war.

Infolge der hohen F-Gehalte der Gläser enthalten sie in merklichem Ausmaß Fluoride, wie z.B. CaF₂. Daher sind die aus den Kationgehalten errechneten Oxidgehalte und demnach der Absolutgehalt des Glases an Sauerstoff zu hoch, und die Summe der Komponentenmenge überschreitet 100 %. Der über 100 % hinausgehende Anteil wird daher üblicherweise als sogenannter "fluoräquivalenter Sauerstoff" ausgewiesen. Dies ist für fluoridhaltige silikatische Gläser üblich und wird z.B. in J. Lange "Rohstoffe der Glasindustrie", Deutscher Verlag für Grundstoffindustrie, Leipzig, Stuttgart (1993), S. 221-223, ausführlich beschrieben.

Es ist in der Glasherstellung allgemein üblich, Fluoride in geringen Mengen als Flußmittel zuzusetzen, um das Schmelzverhalten des jeweiligen Glases zu verbessern. Dies ist auch, wie einleitend beschrieben, bei Gläsern für konventionelle Dentalwerkstoffe bekannt. Durch diese geringen Anteile an Fluor wird jedoch die Gesamtstruktur der Gläser nicht wesentlich verändert.

Demgegenüber ist in dem erfindungsgemäß eingesetzten transparenten Glas ein hoher Fluoranteil von mindestens 4,0 Gew.% eingebaut, der die Grundstruktur des Glases wesentlich gegenüber entsprechenden Gläsern verändert, die frei von Fluor sind oder nur geringe Fluorgehalte infolge eingesetzten Flußmittels haben.

Durch diesen hohen Anteil an Fluor und den gleichzeitigen Einbau von weiteren Netzwerkwandlerionen, wie z.B. Ca²⁺ oder Na⁺, erfolgt ein starker Abbau der SiO₄-Tetraeder-Netzwerkstruktur des Glases. Es entsteht eine Glasstruktur, die mit der klassischen Netzwerktheorie nicht mehr zu erklären ist. Die Glasstruktur nähert sich einer neuen Glasstruktur an, die als "Invertglassstruktur" bezeichnet wird. Unter einem Invertglas versteht man ein Glas, das weniger als 50 Mol-% Netzwerkbildneranteil hat.

Infolge der geänderten Struktur verändert sich vor allem der Brechungsindex des Glases, und es ist überraschenderweise auch eine Fluorionenabgabe bei gleichzeitiger Calciumionenabgabe aus dem Glas möglich. Bei Einsatz des Kompositmaterials im Dentalbereich kann somit in der Mundhöhle durch die Calciumionen zusammen mit Carbonat im Speichel die gewünschte alkalische Wirkung und durch die Fluorionen deren bekannte remineralisierende Wirkung hervorgerufen werden. Auch Calciumionen fördern den Remineralisierungsprozeß.

Weiter bewirkt der hohe Fluoridanteil des Glases eine starke Absenkung von dessen Brechungsindex auf Werte von unter 1,60 und bevorzugt unter 1,56. Die durch Härtung des polymerisierbaren Monomers entstehende organische Matrix des Kompositmaterials hat einen sehr ähnlichen Brechungsindex, weshalb das gesamte Kompositmaterial ebenfalls transluzent oder sogar transparent sein kann. Dies ist von besonderem Vorteil, wenn das Kompositmaterial für die Herstellung von sichtbaren Dentalrestaurationen eingesetzt wird, die naturgemäß ähnliche optische Eigenschaften wie transluzentes natürliches Dentalmaterial haben sollen.

Zur Herstellung des erfindungsgemäß eingesetzten transparenten Glases werden entsprechende Rohstoffe, insbesondere Oxide, Carbonate und Fluoride, gemischt und bei Temperaturen von insbesondere 1000 bis 1600 °C zu einem Glas erschmolzen. Die entstehende Glasschmelze wird dann durch Eingießen in Wasser abgeschreckt. Die erhaltene transparente Glasfritte wird gemahlen und getrocknet und kann dann mit polymerisierbarem Monomer zu dem erfindungsgemäßen polymerisierbaren Kompositmaterial kombiniert werden.

Üblicherweise wird das Glas als Pulver eingesetzt, wobei die mittlere Größe der Teilchen in der Regel 1 bis 100µm und bevorzugt 10 bis 30 µm, bezogen auf die Teilchenanzahl, beträgt.

Neben dem Glas kann das Kompositmaterial auch noch übliche Füllstoffkomponenten enthalten, wie z.B. amorphe kugelförmige Materialien auf Basis von Mischoxiden aus SiO₂, ZrO₂ und/oder TiO₂, mikrofeine Füllstoffe, wie pyrogene Kieselsäure oder Fällungskieselsäure, sowie Makro- oder Minifüllstoffe, wie Quarz-, Glaskeramik- oder Glaspulver mit einer durchschnittlichen Teilchengröße von 0,01 bis 5 µm und schließlich röntgenopake Füllstoffe wie Ytterbiumtrifluorid.

Auch der Einsatz von weiteren Gläsern, die Ionen freisetzen und z.B. für die Herstellung von Glasionomerzementen bekannt sind, ist möglich. Dabei handelt es sich um Glaspulver von üblichen Fluoroaluminosilikatgläsern einer mittleren Teilchengröße von ca. 0,05 bis 15 µm, die als Hauptbestandteile Siliciumoxid, Aluminiumoxid und Calciumoxid enthalten (vgl. A.D. Wilson, J.W. McLean, Glasionomerzement, Quintessenz Verlags-GmbH 1988, Berlin, Seiten 21 ff.).

Das transparente Glas sowie die ggf. vorhandenen weiteren anorganischen Bestandteile des Kompositmaterials können in üblicher Weise silanisiert werden, um den Verbund zwischen ihnen und der organischen Matrix zu verbessern. Als Haftvermittler eignet sich z.B. 3-Methacryloyloxypropyltrimethoxysilan.

Neben dem oben beschriebenen speziellen transparenten Glas mit hoher Calcium- und Fluorionenabgabe enthält das erfindungsgemäße polymerisierbare Kompositmaterial zudem mindestens ein polymerisierbares Monomer. Geeignete Monomere sind die Monomere selbst, daraus hergestellte polymerisierbare Präpolymere sowie Mischungen von diesen. Insbesondere eignen sich als Monomere monofunktionelle oder polyfunktionelle (Meth)acrylate, die alleine oder in Mischungen eingesetzt werden können. Als Beispiele für diese Verbindungen kommen Methylmethacrylat, Isobutylmethacrylat, Cyclohexylmethacrylat, Triethylenglycoldimethacrylat, Diethylenglycoldimethacrylat, Tetraethylenglycoldimethacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, ethoxyliertes Bisphenol-A-dimethacrylat, aber auch Bis-GMA (2,2-bis-4-(3-methacryloxy-2-hydroxypropyl)-phenylpropan) sowie die Reaktionsprodukte aus Isocyanaten, insbesondere Di- und/oder Triisocyanaten und OH-gruppenhaltigen Methacrylaten, und die entsprechenden Acrylate aller obigen Verbindungen in Frage. Beispiele für Reaktionsprodukte von Isocyanaten sind die Umsetzungsprodukte von 1 Mol Hexamethylendiisocyanat mit 2 Mol 2-Hydroxyethylmethacrylat, von 1 Mol (Tri(6-isocyanatohexyl)biuret mit 3 Mol Hydroxyethylmethacrylat und von 1 Mol Trimethylhexamethylendiisocyanat mit 2 Mol Hydroxyethylmethacrylat, die auch als Urethandimethacrylate bezeichnet werden.

Besonders bevorzugt wird als polymerisierbares Monomer ein Mischung von
(i) mindestens einem nicht-aciden, nicht-ionischen, hydrophilen Vernetzermonomer und
(ii) mindestens einem nicht-aciden, nicht-ionischen, hydrophilen Verdünnungsmonomer mit einer Viskosität von kleiner als 1 Pas
verwendet.

Als Vernetzermonomere werden solche Monomere bezeichnet, die mindestens zwei, vorzugsweise 2 bis 4 polymerisationsfähige Gruppen pro Monomermolekül enthalten.

Die Vernetzer- und die Verdünnungsmonomere sind hydrophil, d.h. sie sind zu hydrophilen Wechselwirkungen mit dem Glas in der Lage. Bevorzugt sind Monomere, die eine oder mehrere, vorzugsweise 1 bis 2 Urethan- und/oder OH-Gruppen, vorzugsweise OH-Gruppen enthalten. Es wurde außerdem gefunden, daß diese Gruppen den Ionentransport bzw. die Ionenabgabe aus dem Glas fördern.

Unter nicht-aciden Verbindungen werden Monomere verstanden, die keine stark aciden Gruppen wie Carboxyl-, Phosphorsäure-, Phosphonsäure-, Phosphinsäure- oder Sulfonsäuregruppen tragen und die vorzugsweise auch keine schwach aciden Gruppen wie phenolische OH-Gruppen, SH-Gruppen oder CH-acide Gruppen wie β-Diketon- oder β-Diketoestergruppen enthalten.

Nicht-ionisch im Sinne dieser Erfindung sind Monomere, die keine ionischen Gruppen wie kationische Ammonium- oder Sulfoniumgruppen bzw. anionische Säurerestgruppen der oben genannten stark aciden Gruppen enthalten.

Bevorzugte Vernetzermonomere sind 2,2-Bis-4-(3-methacryloxy-2-hydroxypropyl)-phenylpropan) (Bis-GMA), d.h. das Umsetzungsprodukt von Glycidylmethacrylat und Bisphenol-A (OH-gruppenhaltig), und 7,7,9-Trimethyl-4,13-dioxo-3,14-dioxa-5,12-diazahexadecan-1,16 -diyl-dimethacrylat (UDMA), d.h. das Urethandimethacrylat aus 2 Mol 2-Hydroxyethylmethacrylat (HEMA) und 1 Mol 2-2,4-Trimethylhexamethylendiisocyanat (urethangruppenhaltig). Darüber hinaus sind Umsetzungsprodukte von Glycidylmethacrylat mit anderen Bisphenolen, wie z.B. Bisphenol-B (2,2'-Bis-(4-hydroxyphenyl)-butan), Bisphenol-F (2,2'-Methylendiphenol) oder 4,4'-Dihydroxydiphenyl, sowie Umsetzungsprodukte von 2 Mol HEMA oder 2-Hydroxypropyl(meth)acrylat mit, insbesondere 1 Mol, bekannter Diisocyanate, wie z.B. Hexamethylendiisocyanat, m-Xylylendiisocyanat oder Toluylendiisocyanat, als Vernetzermonomere bevorzugt.

Als Verdünnungsmonomere werden Monomere mit einer Viskosität von < 1 Pas, vorzugsweise < 100 mPas bezeichnet, die sich zur Verdünnung der im allgemeinen hochviskosen Vernetzermonomere eignen und so die Herstellung von Kompositen mit einem hohen Füllstoffgehalt erlauben. Die Viskositätangaben beziehen sich auf eine Temperatur von 23 °C. Die Bestimmung der Viskosiät erfolgt mittels eines Platten- oder Rotationsviskosimeters gemäß DIN 53018.

Die Verdünnungsmonomere enthalten ebenfalls mindestens zwei, vorzugsweise zwei bis drei polymerisationsfähige Gruppen und mindestens eine, vorzugsweise 1 bis 2 OH- und/oder Urethangruppen, vorzugsweise OH-Gruppen.

Ein besonders bevorzugtes Verdünnungsmonomer ist Glycerindimethacrylat (GDMA). Andere bevorzugte Verdünnungsmonomere lassen sich durch Umsetzung von niedrigviskosen Di- oder Triepoxiden, wie beispielsweise Ethylenglykoldiglycidylether, Glycerintriglycidylether oder Trimethylolpropantriglycidylether mit (Meth)-acrylsäure herstellen. Weiter bevorzugt sind darüber hinaus die Umsetzungsprodukte von 2 oder 3 Mol Methacrylsäure mit Glycerintriglycidylether oder Trimethylolpropantriglycidylether. Unter "niedrigviskos" werden Substanzen mit einer Viskosität von < 200 mPas, vorzugsweise < 100 mPas verstanden (23 °C).

Bevorzugte polymerisationsfähige Gruppen sind sowohl bei den Vernetzungs- als auch bei den Verdünnungsmonomeren Methacryl- und/oder Acrylgruppen, insbesondere Methacrylgruppen.

Es hat sich überraschenderweise herausgestellt, daß bei Verwendung einer Mischung von Vernetzermonomer (i) und Verdünnungsmonomer (ii) Komposite erhalten werden, die nicht nur einen hohen Grad an Ionenfreisetzung zeigen, sondern zudem ihre mechanischen Eigenschaften auch nach längerem Kontakt mit Wasser, wie z.B. Lagerung in Wasser, nicht wesentlich verschlechtern. Darüber hinaus sind entsprechende Kompositpasten auch unter feuchter Atmosphäre sehr lagerstabil. Das ungehärtete Kompositmaterial kann bis zu 1,0 Gew.-% Wasser enthalten, ohne daß seine Lagerbeständigkeit oder die mechanischen Eigenschaften des ausgehärteten Materials beeinträchtigt würden. Dies erleichtert sowohl die Herstellung als auch die Verarbeitung durch den Zahnarzt oder Zahntechniker ganz erheblich.

Es ist weiter bevorzugt, daß das Kompositmaterial mindestens 5 Gew.-%, besonders bevorzugt mindestens 10 Gew.-% hydroxylgruppenhaltige Monomere, d.h. Monomere mit mindestens einer Hydroxylgruppe pro Monomermolekül enthält. Es hat sich ebenfalls als vorteilhaft erwiesen, wenn das erfindungsgemäße Material maximal 2 Gew.-% an monofunktionellen Monomeren, d.h. Monomeren mit nur einer ungesättigten, polymerisationsfähigen Gruppe, wie beispielsweise 2-Hydroxyethyl(meth)acrylat, aufweist.

Das erfindungsgemäße Kompositmaterial enthält zudem üblicherweise einen Polymerisationskatalysator (c). Das Kompositmaterial kann je nach Art des verwendeten Katalysators heiß, kalt oder durch Photopolymerisation aushärtbar sein. Kombinationen daraus sind jedoch auch möglich (Dualhärtung).

Als Katalysatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch α,α'-Azo-bis-(isobutyroethylester), Benzpinakol und 2,2'-Dimethylbenzpinakol sind geeignet.

Als Katalysatoren für die bevorzugte Photopolymerisation können z.B. Benzophenon und seine Derivate, Acylphosphinoxide sowie Benzoin und seine Derivate verwendet werden. Beispiele für bevorzugte Photoinitiatoren sind die α-Diketone wie 9,10-Phenanthrenchinon, Diacetyl, Furil, Anisil, 4,4'-Dichlobenzil und 4,4'-Dialkoxybenzil. Campherchinon wird besonders bevorzugt verwendet. Die Verwendung der Photoinitiatoren zusammen mit einem Reduktionsmittel wird bevorzugt. Beispiele für Reduktionsmittel sind Amine wie Cyanethylmethylanilin, Dimethylaminoethylmethacrylat, Triethylamin, Triethanolamin, N,N-Dimethylanilin, N-Methyldiphenylamin und N,N-dimethyl-sym.-xylidin, N,N-Dimethyl-p-toluidin und p-Dimethylaminobenzoesäureethylester. Als Katalystorgemische können die Photoinitiatoren und Reduktionsmittel zusammen mit Katalysatoren für die Heißpolymerisation (bevorzugt mit Peroxiden) eingesetzt werden.

Als Katalysatoren für die Kaltpolymerisation werden Radikale liefernde Systeme, z.B. Benzoyl- bzw. Lauroylperoxid zusammen mit Aminen wie N,N-Dimethyl-sym.-xylidin, N,N-Di-2-hydroxyethyl-p-toluidin oder N,N-Dimethyl-p-toluidin verwendet.

Die in dem Kompositmaterial eingesetzen Mengen an polymerisierbaren Monomeren (a), transparentem Glas (b) und Polymerisationskatalysator zusammen mit ggf. eingesetztem Aktivator (c) sind insbesondere wie folgt:
(a) 15 bis 70 Gew.%, bevorzugt 20 bis 45 Gew.% polymerisierbare Monomere,
(b) 20 bis 85 Gew.%, bevorzugt 40 bis 60 Gew.% transparentes Glas,
(c) 0,01 bis 5,0 Gew.%, bevorzugt 0,1 bis 2,0 Gew.%, Polymerisationskatalysator und ggf. vorhandener Aktivator.

Die Angaben in Gew.% sind auf das Kompositmaterial bezogen.

Das erfindungsgemäße Kompositmaterial wird vorzugsweise als Dentalmaterial eingesetzt. Hierfür spielen seine besonderen Eigenschaften eine Rolle, wobei gerade die hohe Transluzenz sowie die hohe Abgabe von Calcium- und Fluorionen bei Kontakt mit wäßrigen Medium, insbesondere in der Mundhöhle, hervorzuheben ist. Infolge der hohen Transluzenz ist das Kompositmaterial besonders gut für Restaurationsmaterialien geeignet, die nach Härtung dem natürlichen Zahnmaterial ähneln sollen. Überdies erlaubt die hohe Transluzenz die Bereitstellung von ausschließlich lichthärtenden Materialien mit einer sehr hohen Durchhärtungtiefe, so daß sie selbst als Füllungsmaterial für tiefe Kavitäten eingesetzt werden können. Die hohe Ionenabgabe führt schließlich zu einer Inhibierung von Sekundärkaries.

Weiter ist zu betonen, daß das gehärtete erfindungsgemäße Kompositmaterial überraschenderweise selbst bei längerem Kontakt mit Wasser eine nur unwesentliche Veränderung von mechanischen Eigenschaften, wie Festigkeit und E-Modul, erfährt.

Bei Einsatz des Kompositmaterials als Dentalmaterial wird es auf den zu behandelnden Bereich eines natürlichen oder künstlichen Zahnes aufgebracht, ggf. geformt und anschließend durch Polymerisation gehärtet. Ganz besonders bevorzugt wird das Kompositmaterial als restauratives Material, wie Füllungsmaterial für Kavitäten, eingesetzt.

Die Erfindung wird im folgenden anhand von Beispielen näher erläutert.

### Beispiele

### Beispiele 1 bis 16

Es wurden insgesamt 16 transparente Gläser mit hoher Calcium- und Fluorionenabgabe hergestellt, die in dem erfindungsgemäßen Kompositmaterial eingesetzt werden konnten. Zur Herstellung der Gläser wurden entsprechende Oxide, Carbonate und Fluoride homogen zu einem Gemenge gemischt. Dieses Gemenge wurde in einem Platin-Rhodiumtiegel bei einer Temperatur von 1100 bis 1450 °C während einer Homogenisierungszeit von 30 Minuten bis 3 Stunden zu einer Glaschmelze geschmolzen. Dann wurde die Glasschmelze durch Eingießen in Wasser abgeschreckt. Die erhaltene transparente Glasfritte wurde getrocknet und auf die gewünschte Teilchengröße gemahlen.

In der nachstehenden Tabelle I ist die jeweilige chemische Zusammensetzung der einzelnen Gläser angegeben, wobei die Mengen der einzelnen Komponenten in der Weise durch Analyse des Glases bestimmt wurden, wie es weiter ober beschrieben wurde. Weiter sind der Brechungsindex, die Schmelztemperatur sowie das Aussehen des Glases angeführt.

In allen Fällen wurden transparente Gläser mit einem Brechungsindex von < 1,60 erhalten.

Die Beispiele zeigen, daß durch Veränderung der chemischen Zusammensetzung Gläser mit unterschiedlichen Brechungsindices hergestellt werden können. Dadurch kann der Brechungsindex des Glases an den der organischen Matrix des Kompositmaterials angepaßt werden. Auf diese Weise kann durch Kombination der Gläser mit entsprechenden polymerisierbaren Monomeren oder Gemischen davon ein Kompositmaterial hergestellt werden, welches zu einem transluzenten Material aushärtet, das die hohen optischen Anforderungen an ästhetisch ansprechende restaurative Dentalmaterialien erfüllt.

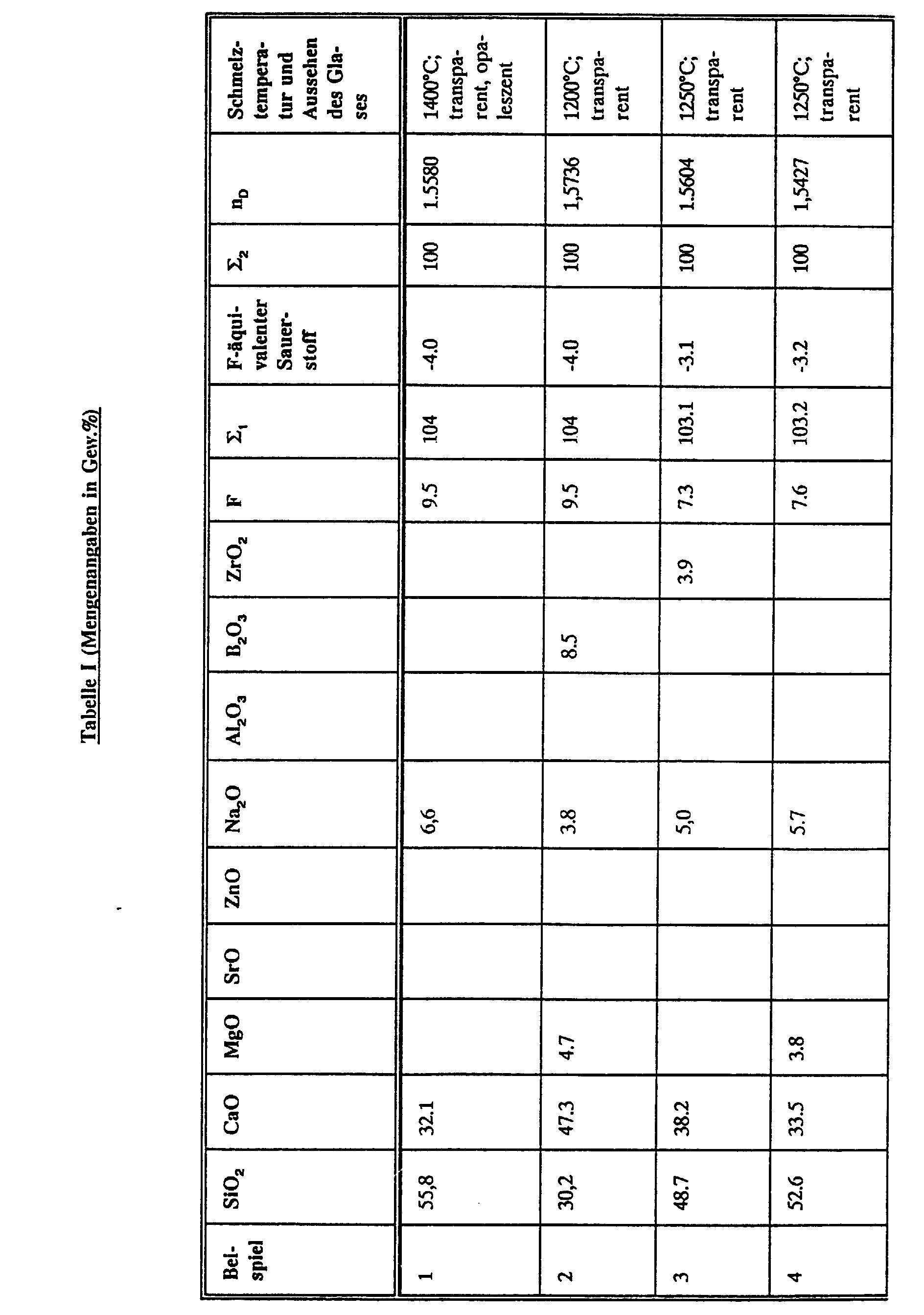

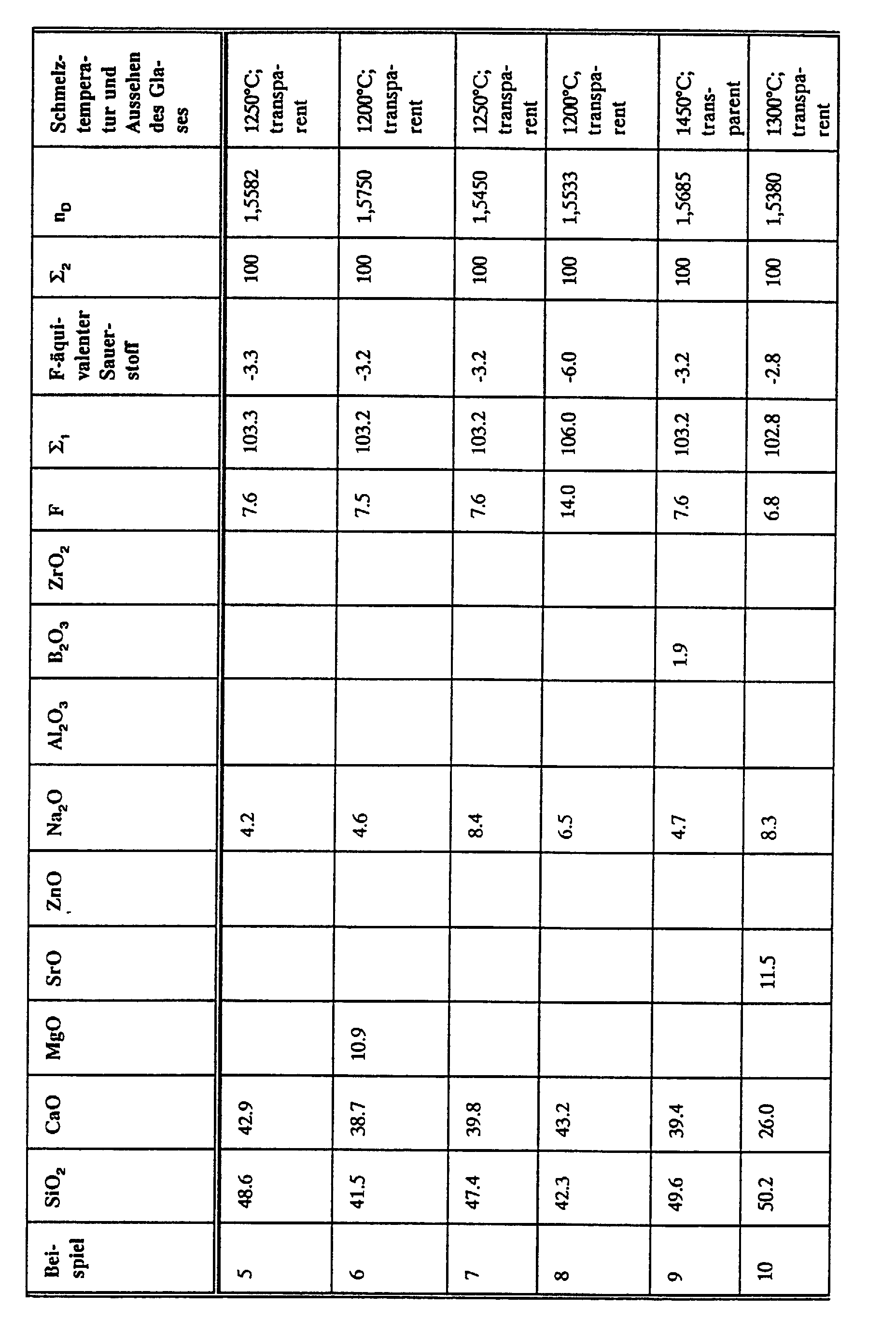

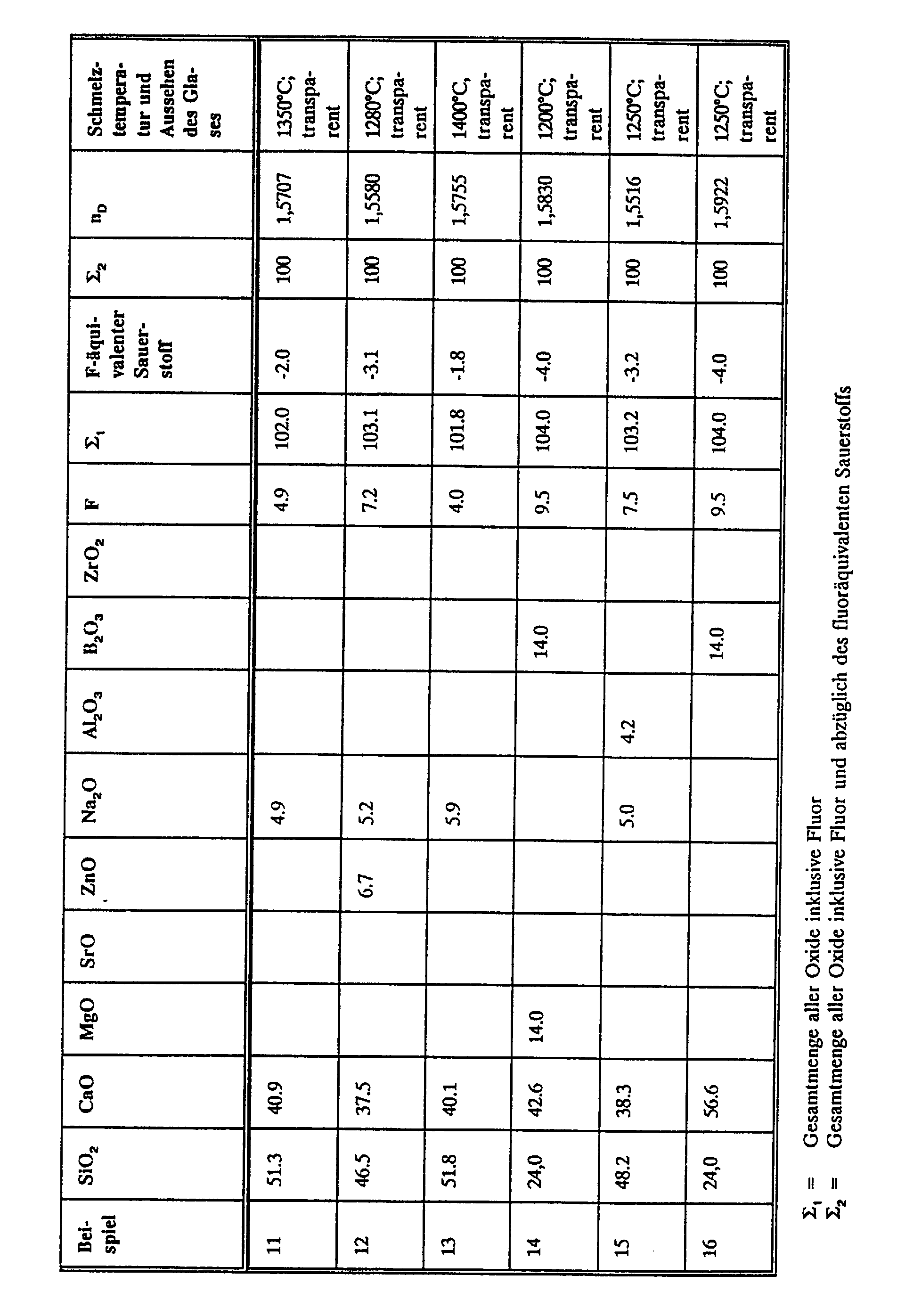

### Beispiel 17

Für die Herstellung von erfindungsgemäßen Kompositmaterialien wurden Monomermischungen der folgenden Zusammensetzung hergestellt.

| **Monomer** | **Mischung (in Gew.%)** | |
|---|---|---|
| | **A** | **B** |
| Bis-GMA ¹⁾ | 39,0 | 42,0 |
| UDMA²⁾ | 30,0 | 37,1 |
| GDMA³⁾ | 30,0 | - |
| TEGDMA⁴⁾ | - | 20,1 |
| Katalysator | 1,0 | 0,8 |

| | | |
|---|---|---|
| ¹⁾ 2,2-bis-4-(3-methacryloxy-2-hydroxypropyl)-phenylpropan | | |
| ²⁾ Urethanmethacrylat erhältlich durch Reaktion von 2 Mol 2-Hydroxyethylmethacrylat und 1 Mol 2,2,4-Trimethylhexamethylendiisocyanat | | |
| ³⁾ Glycerindimethacrylat | | |
| ⁴⁾ Triethylenglycoldimethacrylat | | |

Mit den beiden hergestellten Monomermischungen A und B wurden entsprechende Kompositpasten A und B durch inniges Vermengen mit erfindungsgemäß eingesetztem transparenten Glas sowie weiteren Füllstoffen hergestellt. Die Kompositpasten hatten die aus der folgenden Tabelle ersichtliche Zusammensetzung.

| **Komponente** | **Komposit (in Gew.%)** | |
|---|---|---|
| | **A** | **B** |
| Monomermischung | 22,0 | 22,1 |
| Transparentes Glas¹⁾ | 48,0 | 52,2 |
| SP-2034, sil.²⁾ | 11,0 | - |
| YbF₃ | 12,0 | 10,0 |
| Aerosil-OX-50 sil.³⁾ | 4,0 | 3,8 |
| HDK-2000⁴⁾ | 3,0 | 2,4 |
| Ba-Glas sil. (GM 27884)⁵⁾ | - | 9,5 |

| | | |
|---|---|---|
| ¹⁾ Glas gemäß Beispiel 7 aus Tabelle I, welches in üblicher Weise silanisiert wurde. | | |
| ²⁾ Silanisiertes Fluor-Calcium-Aluminium-Silikatglas. | | |
| ³⁾ Silanisierte pyrogene Kieselsäure (Degussa, Hanau) | | |
| ⁴⁾ Hochdisperse Fällungskieselsäure (Wacker, Burghausen) | | |
| ⁵⁾ Silanisiertes Barium-Aluminium-Silikatglas (Schott, Landshut) | | |

Aus den beiden Kompositpasten wurden zur Bestimmung der mechanischen Eigenschaften und der Fluoridfreisetzung Prüfkörper hergestellt, die zweimal 3 Minuten lang mit dem Licht einer üblichen Dentalpolymerisationslampe, nämlich Spectramat der Ivoclar AG, Liechtenstein, ausgehärtet wurden.

### KOMPOSIT A:

Mechanische Eigenschaften:

| | | |
|---|---|---|
| a) Biegefestigkeit | 24 h H₂O-Lagerung | 122MPa |
| | 6 d H₂O-Lagerung+24 h Kochen | 117 MPa |
| b) Biege-E-Modul | 24 h H₂O-Lagerung | 11,4 GPa |
| | 6 d H₂O-Lagerung+24 h Kochen | 11,3 GPa |

Die Biegefestigkeit und das Biege-E-Modul wurden gemäß 150-Norm 4049 (1988) bestimmt.

### Freisetzung von F-Ionen (kumulativ):

Nach 28 Tagen (Lactat-Puffer, 37 °C): 218 µg/cm⁻²

Zur Ermittlung des Fluoridfreisetzungsvermögens wurden ausgehärtete Prüfkörper (Durchmesser = 20 mm, Höhe = 1,5 mm) in 30 ml Lactat-Pufferlösung bei 37°C im Schüttler gelagert, und nach bestimmten Zeitabständen wurde die freigesetzte Fluoridmenge mit einer Fluorelektrode gemessen.

Im Vergleich dazu zeigt das Komposit B unter gleichen Bedingungen eine Fluoridfreisetzung von 124 µg/cm⁻². Diese etwas verringerte, aber nach wie vor hohe Fluorid-Abgabe rührt daher, daß bei Komposit B anstelle des besonders bevorzugten hydrophilen Glycerindimethacrylats als Verdünnungsmonomer das hydrophobe Triethylenglycoldimethacrylat eingesetzt wurde.

Weiter wurden zum Vergleich die Fluorionen-Abgabe von einem handelsüblichen Kompomer, nämlich Compoglass® von Ivoclar AG, Liechtenstein, und einem handelsüblichen Glasionomerzement, nämlich Vivaglass Fil von Ivoclar AG, Liechtenstein, bestimmt. Diese betrugen ca. 22 µg/cm⁻² für Compoglass und ca. 240µg/cm⁻² für Vivaglass Fil.

Dieser Vergleich belegt, daß das erfindungsgemäße Kompositmaterial auch herkömmlichen Kompomeren in der Fluorionen-Abgabe überlegen ist und ähnliche Werte wie die in dieser Hinsicht besonders leistungsfähigen Glasionomerzemente erreicht.

## Patentansprüche

1. Polymerisierbares Kompositmaterial, dadurch gekennzeichnet, daß es
(a) mindestens ein polymerisierbares Monomer und
(b) mindestens ein transparentes Glas mit hoher Calcium- und Fluorionenabgabe aufweist, welches die folgenden Komponenten enthält:
| Komponente | Gew.-% |
|---|---|
| | |
| SiO₂ | 24,0 bis 56,0 |
| CaO | 26,0 bis 57,0 |
| F | 4,0 bis 14,0. |

2. Kompositmaterial nach Anspruch 1, dadurch gekennzeichnet, daß das Glas zusätzlich mindestens eine der folgenden Komponenten enthält
| Komponente | Gew.-% |
|---|---|
| | |
| Na₂O | 1,0 bis 9,0 |
| B₂O₃ | 1,0 bis 14,0 |
| MgO | 1,0 bis 14,0 |
| SrO | 1,0 bis 12,0 |
| ZnO | 1,0 bis 7,0 |
| Al₂O₃ | 0,5 bis 5,0 |
| ZrO₂ | 0,5 bis 4,0. |

3. Kompositmaterial nach Anspruch 1 oder 2, dadurch gekennzeichnet, daß die Komponenten des Glases unabhängig voneinnder in den folgenden Mengen vorhanden sind
| Komponente | Gew.-% |
|---|---|
| | |
| SiO₂ | 30,0 bis 54,0, insbesondere 36,0 bis 54,0 |
| CaO | 32,0 bis 50,0 |
| F | 5,0 bis 12,0 |
| Na₂O | 1,0 bis 8,0 |
| B₂O₃ | 1,0 bis 12,0 |
| MgO | 1,0 bis 10,0 |
| SrO | 1,0 bis 10,0 |
| ZnO | 1,0 bis 5,0 |
| Al₂O₃ | 0,5 bis 4,0 |
| ZrO₂ | 0,5 bis 4,0. |

4. Kompositmaterial nach einem der Ansprüche 1 bis 3, dadurch gekennzeichnet, daß die Komponenten des Glases unabhängig voneinander in den folgenden Mengen vorhanden sind
| Komponente | Gew.-% |
|---|---|
| | |
| SiO₂ | 45,0 bis 54,0 |
| CaO | 35,0 bis 50,0 |
| F | 6,0 bis 12,0 |
| Na₂O | 4,0 bis 7,0 |
| B₂O₃ | 1,0 bis 12,0 |
| MgO | 1,0 bis 10,0 |
| SrO | 1,0 bis 10,0 |
| ZnO | 1,0 bis 5,0 |
| Al₂O₃ | 0,5 bis 4,0 |
| ZrO₂ | 0,5 bis 4,0. |

5. Kompositmaterial nach einem der Ansprüche 1 bis 4, dadurch gekennzeichnet, daß das Glas einen Brechungsindex von weniger als 1,60 und insbesondere weniger als 1,56 hat.

6. Kompositmaterial nach einem der Ansprüche 1 bis 5, **dadurch gekennzeichnet**, daß es eine Mischung von
(i) mindestens einem nicht-aciden, nicht-ionischen, hydrophilen Vernetzermonomer und
(ii) mindestens einem nicht-aciden, nichtionischen, hydrophilen Verdünnungsmonomer mit einer Viskosität von weniger als 1 Pas
enthält.

7. Kompositmaterial nach einem der Ansprüche 1 bis 6, **dadurch gekennzeichnet**, daß es mindestens 5 Gew.-% hydroxylgruppenhaltige Monomere enthält.

8. Kompositmaterial nach Anspruch 6 oder 7, **dadurch gekennzeichent**, daß es als Vernetzermonomer 2,2-Bis-4-(3-methacryloxy-2-hydroxypropyl)-phenylpropan) (Bis-GMA), 7,7,9-Trimethyl-4,13-dioxo-3,14-dioxa-5,12-diazahexadecan-1,16-diyl-dimethacrylat (UDMA), ein Umsetzungsprodukt von Glycidylmethacrylat mit einem Bisphenol und/oder ein Umsetzungsprodukt von 2 Mol 2-Hydroxyethylmethacrylat (HEMA) oder 2-Hydroxypropyl(meth)acrylat mit 1 Mol Diisocyanat enthält.

9. Kompositmaterial nach einem der Ansprüche 6 bis 8, **dadurch gekennzeichnet**, daß es als Verdünnungsmonomer Glycerindimethacrylat (GDMA), ein Umsetzungprodunkt von niedrigviskosen Di- und Triepoxiden mit (Meth)acrylsäure und/oder ein Umsetzungsprodukt von 2 oder 3 Mol Methacrylsäure mit Glycerintriglycidylether oder Trimethylolpropantriglycidether enthält.

10. Transparentes Glas mit hoher Calcium- und Fluorionenabgabe zur Verwendung als Füllstoff in Dentalmaterialien, **dadurch gekennzeichnet**, daß es die folgenden Komponenten enthält
| Komponente | Gew.-% |
|---|---|
| | |
| SiO₂ | 24,0 bis 56,0 |
| CaO | 26,0 bis 57,0 |
| F | 4,0 bis 14,0. |

11. Verwendung des Kompositmaterials nach einem der Ansprüche 1 bis 9 als Dentalmaterial.

12. Verwendung nach Anspruch 11, wobei das Dentalmaterial ein Füllungsmaterial für Kavitäten ist.
